# EUROPEAN PATENT SPECIFICATION

(11) **EP 4 182 894 B1**
(45) Date of publication and mention of the grant of the patent: **19.11.2025**
(21) Application number: 21786795.1
(22) Date of filing: 28.09.2021
(51) Int. Cl.: G06V 10/25, G06V 10/82

(54) **METHOD AND SYSTEM FOR AUTOMATIC DETECTION OF FREE INTRA-ABDOMINAL AIR**
VERFAHREN UND SYSTEM ZUR AUTOMATISCHEN ERKENNUNG VON FREIER INTRAABDOMINALER LUFT
PROCÉDÉ ET SYSTÈME DE DÉTECTION AUTOMATIQUE D'AIR INTRA-ABDOMINAL LIBRE

(30) Priority: 01.10.2020 EP 20199641
(43) Date of publication of application: 24.05.2023
(73) Proprietor: Siemens Healthineers AG, 91301 Forchheim (DE); Herlev and Gentofte Hospital, 2730 Herlev (DK)
(72) Inventor: TAUBMANN, Oliver, 91365 Weilersbach (DE); BREJNEBØL, Mathias Willadsen, 2100 København Ø (DK); MÜLLER, Christoph Felix, 2200 Copenhagen (DK); EIBENBERGER, Eva, 90427 Nürnberg (DE); SÜHLING, Michael, 91052 Erlangen (DE)
(74) Representative: Siemens Healthineers Patent Attorneys
(86) International application number: PCT/EP2021/076602
(87) International publication number: WO 2022/069448

(56) References cited:
- CN-A- 111 192 356
- US-A1- 2016 364 857
- US-A1- 2021 201 066

## Description

The present invention relates to a method and system for the detection of free intra-abdominal air.

The present invention is in the field of medical imaging, which is used for of creating visual representations of the interior of a body for clinical analysis and medical intervention. In medical imaging, by using imaging modalities such as computed tomography (CT), magnetic resonance imaging (MR), ultrasound (US) and others, 3D image data and 2D image data representing interior structures of a patient or subject can be obtained and displayed. The image data obtained from scanning a patient or subject nowadays are available in digital formats and are suitable for digital processing, analysis and modification.

Emergency / trauma imaging is an important topic for medical imaging as the extremely time-critical nature of the associated clinical pathway requires solutions that are tightly integrated with the scan workflow.

In the field of abdominal emergency imaging, the identification of pneumoperitoneum (i.e., free intra-abdominal air), a potentially critical condition that may require immediate surgery, is of high clinical interest. If it were possible to detect this condition automatically based on a CT scan, such patients could be automatically prioritized for reading above others suspected to be less critical (triage). Moreover, if this detection were performed directly at the acquisition workplace, it would also enable the scanner to automatically notify the user of such a critical finding. Such a system would be a key component in realizing the vision of an intelligent CT scanner.

The Document CN111192356A discloses a method for displaying a region of interest.

The peritoneum is a membrane that tightly surrounds the majority of the ab dominal organs. The folds of the peritoneum create an intra-abdominal space, the peritoneal cavity. During the diagnosis of patients with severe abdominal pain, the identification of air inside the peritoneal cavity (pneumoperitoneum) is an important and time-critical task for radiologists. The most common non-surgical cause of pneumoperitoneum is a perforation of the viscus. More precisely, free intra-abdominal air indicates the rupturing of a hollow organ, mostly of the gastrointenstinal tract. Such a rupture is a surgical emergency and requires immediate intervention in order to prevent contamination within the peritoneal cavity [9, 10]. As the perforation itself often cannot be recognized directly with the imaging modalities commonly used in emergency care, the air acts as a surrogate radiological sign. Computed tomography (CT) is considered to be the standard for discrimination of extraluminal and intraluminal gas [1, 5, 9]. However, depending on the location and amount of free intra-abdominal air-ranging from sub-centimeter air bubbles in close proximity to the bowel wall to large quantities that freely move within the peritoneal cavity-its diagnosis can be challenging and inconspicuous cases might be missed, especially when there is no initial suspicion of pneumoperitoneum. In addition, other cases may occupy the higher-priority spots of the emergency reading worklist, causing easily identifiable cases of pneumoperitoneum to go unnoticed until the scan is read at a later time, at which the patient may already have faced serious consequences. An automatic detection algorithm would be useful [13] for both scenarios, i. e. the identification as well as the prioritized reading (triage) of potentially incidentally found cases of pneumoperitoneum. While prior work has addressed detection of pneumoperitoneum on chest X-rays [7], no method for the automatic detection of free intra-abdominal air on CT scans is available.

It is thus an object of the invention to provide improved methods and systems for the detection of free intra-abdominal air.

This object is achieved by the invention according to the independent claims.

In the following the solution according to the invention is described with respect to systems as well as with respect to methods. Elements, characteristics, advantages or alternative embodiments herein can be assigned to the other claimed objects and vice versa. In other words, claims for the providing systems can be improved with features described or claimed in the context of the methods, and vice versa. In the former case, the functional features of the method are embodied by objective units of the providing system. Furthermore, elements, characteristics, advantages or alternative embodiments described in connection with particular exemplary embodiments can be assigned to the invention in its most general terms.

In its most general terms, the invention relates to a method, preferably a computer implemented method, for providing out-put data. The method of the invention, in the most general terms comprises the steps of
- receiving input data, e.g. via a first interface,
- generating output data by applying algorithmic operations to the input data,
- providing the output data, e.g. via a second interface.

In its most general terms, the invention further relates to a system, comprising
- a first interface, configured for receiving input data,
- a second interface, configured for providing output data,
- a computation unit, configured for applying algorithmic operations to the input data, wherein the output data is generated.

Said computation unit may optionally comprise a plurality of calculation units for performing separate algorithmic operations. Said plurality of calculation units may be provided as independent, connected subunits of the computation unit or, in the alternative, may be provided as one integrated computation unit.

In its most general terms, the invention further relates to a computer program comprising instructions which, when the program is executed by a computer cause the computer to carry out the methods of invention.

In its most general terms, the invention further relates to a computer-readable medium comprising instructions which, when executed by a computer cause the computer to carry out the methods of invention.

Most of the aforementioned units, can be implemented in full or in part in the form of software modules in a processor of a suitable control device or of a processing system. An implementation largely in software has the advantage that even control devices and/or processing systems already in use can be easily upgraded by a software update in order to work in the manner according to at least one embodiment of the invention.

The term "unit" may be replaced with the term "circuit" or "module". The term "unit" may refer to, be part of, or include processor hardware (shared, dedicated, or group) that executes code and memory hardware (shared, dedicated, or group) that stores code executed by the processor hardware.

The units may be implemented in one control unit and each unit may include one or more interface circuits. Alternatively, the units may be implemented in a delocalized system, e.g. in a network. In some examples, the interface circuits may include wired or wireless interfaces that are connected to a local area network (LAN), the Internet, a wide area network (WAN), or combinations thereof. The functionality of any given unit of the present disclosure may be distributed among multiple modules that are connected via interface circuits. For example, multiple modules may allow load balancing. In a further example, a server (also known as remote, or cloud) module may accomplish some functionality on behalf of a client module.

In particular, the invention relates to a (e.g. computer-implemented) method for the detection of free intra-abdominal air, comprising:
- receiving input data, said input data comprising a medical imaging data set of an abdominal region of a patient, e.g. via a first interface
- applying a trained function, wherein the output data is generated,
- providing the output data e.g. via a second interface,
wherein the medical imaging data set comprises 3D imaging data and wherein the 3D imaging data is subdivided into a plurality of image patches of a preselected size, wherein the trained function is used to determine whether any given image patch the plurality of image patches contains free intra-abdominal air, and
wherein a classification information or score or probability information is determined for each image patch.

In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Further-more, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Further-more, a neural network can be an adversarial network, a deep adversarial network and/or a generative adversarial network.

The output data can comprise a flag or indication of the medical imaging data set indicating the presence or absence of free intra-abdominal air. Such flag or indication can comprise a visual marker or an acoustic marker. The output data can further comprise a marker for highlighting an image region comprising free intra-abdominal air in a displayed image based on or derived from the medical imaging data set. The output data can comprise a flag or indication for free intra-abdominal in a given subregion of the medical imaging data set or in a given subregion of a displayed image based on or derived from the medical imaging data set. The output data can comprise a classification information indicating either presence or absence of free intra-abdominal air. The output data can comprise a score or probability information indicating a probability or likelihood of presence or absence of free intra-abdominal air. Such classification information or score or probability information may relate to the medical imaging data set as a whole or to a given subregion of the medical imaging data set or to a given subregion of a displayed image based on or derived from the medical imaging data set. The output data can be displayed, stored or processed further. The output data can be transmitted to a hospital information system (HIS) or radiology information system (RIS). The output data can be utilized to trigger further patient exams or diagnostic procedures.

The trained function can, for example, be trained on training data comprising a plurality of medical imaging data sets of an abdominal region, wherein image regions containing free intra-abdominal air are annotated.

The annotation of image regions containing free intra-abdominal air can, for example, comprise placing markers manually that indicate positions inside the air regions. In other words, annotation of an image region containing free intra-abdominal air can, for example, comprise placing a marker manually that indicates a position inside a region of free intra-abdominal air.

The medical imaging data set can, for example, comprise a CT imaging data set. The medical imaging data set may comprise a raw data set, a reconstructed image data set, and/or an image data set which has been further processed after reconstruction. The medical imaging data set may comprise further data, such as segmentation data and data derived from segmentation data, such as identification data related to a segmented structure within the medical imaging data set. Image segmentation is the process of partitioning a digital image into multiple segments (sets of pixels, also known as image objects). The goal of segmentation is to simplify and/or change the representation of an image into something that is more meaningful and easier to analyze, and allows the analysis of anatomic structure, such as blood vessels.

Optionally, the medical imaging data set can, for example, be further processed to define a search space. The search space is a subregion of the imaging data to which the trained function is applied. Such further processing can be performed manually or automatically, for example by segmentation of selected anatomical structures which may be either included n or excluded from the search space. Applying the trained function to a search space increases the efficiency and speed of the method, as non-relevant areas of the images to be analyzed are disregarded.

The medical imaging data set comprises 3D imaging data, wherein the 3D imaging data is subdivided into a plurality of image patches of a preselected size. The image patches can be adjacent or overlapping.

The trained function is applied to the plurality of image patches to determine whether any given image patch contains free intra-abdominal air. This is an example of a sliding window approach.

In other words, a classification information or score or probability information is determined for each image patch. To make an overall determination for the patient (in other words with regard to the entire medical imaging data set, or at least the entire search space) the output data can comprise a classification information or score or probability information based on the N image patches with the Nth highest scores or probability information.

The medical imaging data set can, for example, be further subdivided into a first plurality of image patches of a first preselected size and into second plurality of image patches of a second preselected size. The image patches of the first and second plurality of image patches can, for example, overlap. The trained function can then be applied to determine whether any given image patch of the first and the second plurality of image patches contains free intra-abdominal air. This will result in a more robust and improved classification or analysis of the medical imaging data set as a whole.

The trained function can, for example, be based on a convolutional neural network or deep neural network.

In particular further, the invention relates to a system or apparatus for the detection of free intra-abdominal air according to the methods of the invention for the detection of free intra-abdominal air as defined in the patent claims, comprising
- a first interface, configured for receiving input data,
- a second interface, configured for providing output data,
- a computation unit, configured for applying a trained function, wherein the output data is generated.

The system or apparatus could be used as a second reader to help avoid cases of pneumoperitoneum being missed accidentally.

In particular further, the invention relates to a computer program comprising instructions which, when the program is executed by a computer to carry out the methods for the detection of free intra-abdominal air of the invention.

Further, the invention relates to a computer-readable medium comprising instructions which, when executed by a computer to carry out the method of the invention for detection detection of free intra-abdominal air.

Further, the invention relates to a (e.g. computer-implemented) method for providing a trained function for the detection of free intra-abdominal air, comprising:
- receiving first input training data (e.g. with a first training interface) comprising a first plurality of medical imaging data sets of an abdominal region, wherein the medical imaging data sets of the first plurality of medical imaging data sets do not comprise image regions containing free intra-abdominal air
- receiving second input training data (e.g. with a second training interface) comprising a second plurality of medical imaging data sets of an abdominal region, wherein each medical imaging data set of the second plurality of medical imaging data sets comprises at least one image region containing free intra-abdominal air.
- training a function based on the input training data and the out-put training data (e.g. with a training computation unit),
- providing the trained function (e.g. with a third training interface),

wherein the medical imaging data sets of the first and second input training data comprise 3D imaging data and wherein the 3D imaging data is subdivided into a plurality of image patches of a preselected size,
wherein the trained function is trained to determine whether any given image patch the plurality of image patches contains free intra-abdominal air, and wherein a classification information or score or probability information may be determined for each image patch.

The medical imaging data set of the second plurality of medical imaging data sets can, for example, comprise annotation information indicating the at least one image region containing free intra-abdominal air.

The annotation information can be provided as yes/no or 1;0 classification information indicative of the presence or absence of free intra-abdominal air. The annotation information can be provided as score or probability information, indicating a probability or likelihood of presence or absence of free intra-abdominal air.

The trained function can, for example, be based on a convolutional neural network or deep neural network.

The medical imaging data sets of the first and second input training data comprises 3D imaging data, wherein the 3D imaging data is subdivided into a plurality of image patches of a preselected size. The image patches can be adjacent or overlapping.

The annotation information can be provided manually by a user, e.g. a radiologist. The annotation information can also be provided in a semiautomatic fashion, wherein a user places a marker inside a region of free intra-abdominal air and through a further automatic processing step, the whole region containing the marker is segmented. From the annotation, further information can be automatically derived, such as an area or volume in the image data comprising free air or a location information relative to the patient's anatomy. Such further information can be included in the output data.

After providing annotation information for a training data set, the training data set can, be subdivided into a plurality of image patches wherein image patches comprising part of an annotated region of free intra-abdominal air are annotated as comprising free intra-abdominal air. Again, such an annotation information may comprise a (binary) classification information or a score or probability information.

Further, the invention relates to a training system for training a function for the detection of free intra-abdominal air, comprising
- a first training interface, configured for receiving input training data,
- a second training interface, configured for receiving output training data, wherein the input training data is related to the output training data,
- a training computation unit, configured training a function based on the input training data and the out-put training data,
- a third training interface, configured for providing the trained function.

Further, the invention relates to a computer program comprising instructions which, when the program is executed by a computer, cause the computer to carry out any of the training methods of the invention.

Further, the invention relates to a computer-readable medium comprising instructions which, when executed by a computer, cause the computer to carry out any of the training methods of the invention.

Further details and advantages of the current invention can be taken from the following description of exemplary embodiments in conjunction with the drawings in which:
Fig. 1 shows a schematic representation of an exemplary embodiment of the invention with a baseline convolutional neural network architecture for sliding-window detection, which maps each 3-D patch to a binary (sigmoid) output variable stating a probability for whether the patch contains free intra-abdominal air;
Fig. 2 shows a diagram of case-level AUCs for a baseline model for different values of N, i. e. how many of the highest individual patch predictions are used to determine the case-level prediction;
Fig. 3 (a)-(c) shows a diagram of Case-level ROC curves of one exemplary CV run for the recurrent model and (d) shows a histogram of the distribution of ground truth markers from which the sub-groups are determined;
Fig. 4 shows a schematic representation of an embodiment of the method of the invention;
Fig. 5 shows a schematic representation of an embodiment of the system of the invention; and
Fig. 6 shows a schematic representation of an embodiment of the training method of the invention.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

The method of the invention applies a learning-based approach to design a system for automatic detection of pneumoperitoneum. It can be trained on a database of CT images of patients and controls with expert annotations indicating the regions of free intra-abdominal air. Afterwards, it is able to identify pneumoperitoneum on unseen scans of new patients with high accuracy.

In an exemplary embodiment of the inventive method, the detection task is approached using a conventional sliding-window approach relying on deep-learning based classification of 3-D image patches. Two further variants/embodiments to enhance this approach employing multi-scale inputs and recurrent learning are disclosed in order to increase the spatial context of the input patch without compromising the association between the label and the location of the finding that caused it. In addition to the default setup that requires only trivial preprocessing, results are shown for a variant or exemplary embodiment that relies on a prior segmentation of the abdominopelvic cavity being available.

To assess the performance of the method while also taking into account the heterogeneity and varying difficulty of the cases, sub-group analysis is performed based chiefly on the amount of free intra-abdominal air observable in the scan.

To this end, one particular embodiment might employ a sliding-window classification approach with a deep neural network as the classifier model, potentially also using multi-scale or recurrent learning.

Other conceivable embodiments may treat the problem as a segmentation task and thus also enable to output, e.g., the amount/volume of free air found, as output data.

The implementation of such an approach could be run directly on the scanner hardware. On the one hand, this means that the presentation of its results can be integrated into the scanner UI, such as in the form of an immediate notification. On the other hand, this also entails the advantage that specific reconstructions best suited to the (automatic) detection of pneumoperitoneum can be performed and used to run the algorithm, whereas a scanner-independent solution would need to rely on whichever images happen to be available.

In particular, the algorithm might prefer an axial thin-slice reconstruction with a relatively sharp kernel so ensure good visibility of small air bubbles and the bowel walls separating intra- from extra-luminal air. Also, information derived from multi-spectral data acquired by dual-energy or photon-counting CT scanners could further improve the detection accuracy, e.g. by allowing to both standardize both overall image contrast (Mono+) and remove unwanted differences caused by iodinated contrast agents (VNC). This is especially relevant because determining whether a volume of air is free requires a good understanding of surrounding anatomy, which in turn strongly relies on soft-tissue contrast.

For example, the input data for the method of the invention can be any abdominal CT scan. Further, for example, the following preprocessing steps can be carried out: The image volume is first resampled in z to obtain a standardized slice thickness of 3 mm. Next, a simple body segmentation is performed to obtain a binary mask of the image region occupied by the patient, which will serve as the search space unless explicitly stated otherwise. Alternatively, the search space can be restricted further by using a segmentation of the abdominopelvic cavity instead, which is obtained from a deep-learning model originally described by Yang et al. for liver segmentation [15].

### Sliding-Window Detection Approach

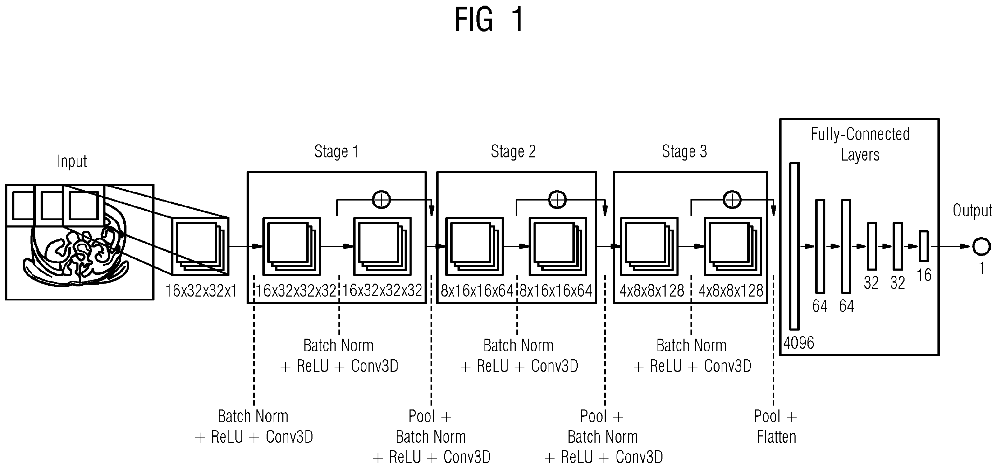

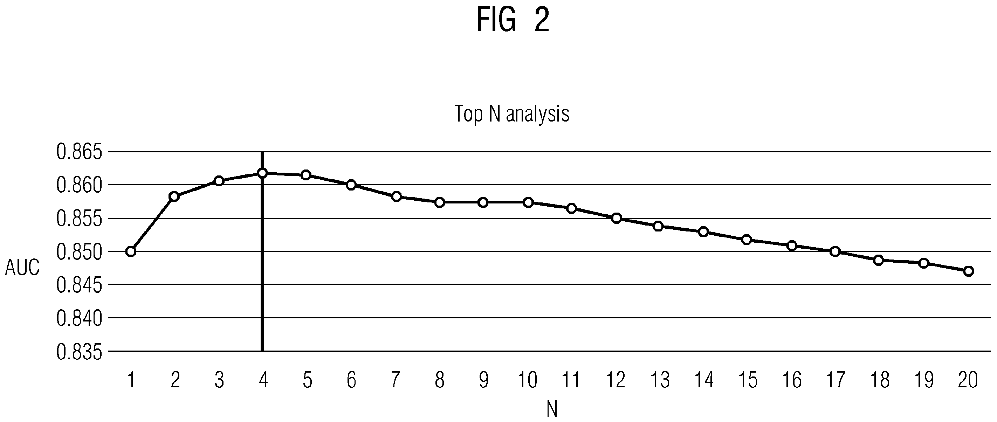

Inference Workflow. Subsequently, and as illustrative example, the volume is subdivided into regular 3-D patches of size (sx, sy, sz) = (64, 64, 16) pixels with 25 % overlap in all dimensions. The patches are downsampled in the axial plane to a size of (sx', sy', sz) = (32, 32, 16) pixels and pixel intensities are normalized. Each patch is then separately fed to the convolutional neural network classifier depicted in Fig. 1. The model is trained to classify whether the patch contains free intra-abdominal air or not. The case-level prediction for a patient is then determined as the average of the output probability scores of the top N patches, i. e. the N boxes that
achieved the highest probabilities, where N = 4 is determined empirically once and then kept fixed (cf. Fig. 2). Only patches that have overlap with the search space are considered. Below, this model will be referred to as the baseline model. Model Training. The ground truth annotation consists in markers placed manually by radiologists that indicate positions inside the air regions. (For a brief discussion of the chosen mode of annotation, please see the section on annotation below.) If an image patch contains any markers, it is labeled as containing free intra-abdominal air, and vice versa. In order to avoid patches with off-center detections while at the same time ensuring complete coverage of all markers, a margin of half the size of the overlap region is excluded for this process of determining the labels. For training, mini batches of size 32 are augmented on the fly with random translations, small-angle rotations and flips, then fed to an Adam optimizer [6] to minimize binary cross-entropy on the training data. Frequency weighting is utilized to cope with the label imbalance. Early stopping is used with a patience of 30 epochs monitoring Matthews correlation coefficient [8] calculated on validation data.

### Enhancing Spatial Context

The obvious downside of sliding window classifiers is the limited spatial context available to the model. However, there is a trade-off involved that prevents one from the trivial solution of choosing ever larger patches to overcome this limitation: With more context, the association between the label and the finding that caused it grows weaker. As an extreme example, consider the case of whole-volume classification: While technically all information is present, even deep learning usually has a hard time identifying the relevant structures due to the enormous input space, unless training data on the order of thousands of cases is available. Below, two exemplary ways to cope with this trade-off are presented.

### Multi-Scale Variant

This exemplary embodiment is a simple extension that borrows its core idea from multi-scale/resolution analysis [14]. For each patch, a second patch centered at the same position, but with twice the (physical) size within the axial plane is extracted. It is downsampled to the same size in pixels as the original patch such that it has half its resolution. This second patch is fed to the model as a separate input and goes through a duplicated (but not parameter-tied) version of the convolutional stages of Fig. 1, with the resulting feature maps of both legs being concatenated before the first fully-connected layer. All other aspects of the training procedure are kept the same.

### Recurrent Variant

Inspired loosely by approaches common in text recognition [12], this exemplary embodiment is a way for the model to incorporate information from previous and subsequent patches by using recurrent units. More precisely, all sliding-window patches with the same position along the axial direction, i. e. a z-slice of 3-D patches which corresponds to an axial slab in the input volume, are treated as a sequence. Within the sequence, the patches are arranged to follow a zig-zag order (first line left-to-right, second line right-to-left, etc.) in order to avoid "jumps." Then a model is designed to bi-directionally traverse such patch sequences and map them to the binary sequences of their labels. In order to have fixed-length sequences, the patches outside the search space are included as well and their predictions after inference are zeroed. As training this model from scratch proves to be challenging, a transfer learning approach is adopted by taking the baseline model described in section 2.2, freezing the trained weights and adding three layers of gated recurrent units (GRU) [2] after the first, third and fifth dense layer, each with the same output tensor dimensions as the preceding layer. These are then trained for ten more epochs, effectively fine-tuning the model to the sequence representation.

### Data

Abdominal CT scans from a total of Npat = 139 patients were selected for a retrospective study, of which Nair = 110 were consecutive pneumoperitoneum cases, while the remaining Nctrl = 29 were controls. The control cases are equivalently acquired scans of other emergency patients with an abdominal pathology that subsequently required surgery, rendering them directly comparable to those of the pneumoperitoneum patients as well as more difficult to distinguish from them than healthy individuals. For training and evaluation purposes, all Npat patients are pooled and five random repetitions of five-fold cross validation (CV) are performed to achieve reliable performance estimates. From four out of five CV subsets used for training, one is set aside as the validation set.

### Figures of Merit

Receiver-operating characteristic (ROC) curve analysis [4] is carried out in a micro-averaging fashion, i. e. with all out-of-sample predictions for one CV repetition combined in one curve, for two classification tasks:
a) Patch-wise binary classification of all 3-D patches of all patients (window-level evaluation) and b) patient-wise binary classification according to the top-4 weighting procedure of patch predictions as described under case-level evaluation, above. While the latter represents a clinically more meaningful assessment,
the former allows for a more fine-grained analysis of the model performance due to the much larger number of samples. In both evaluations, the same training/test splits were performed on the patient level.

### Sub-group Analysis

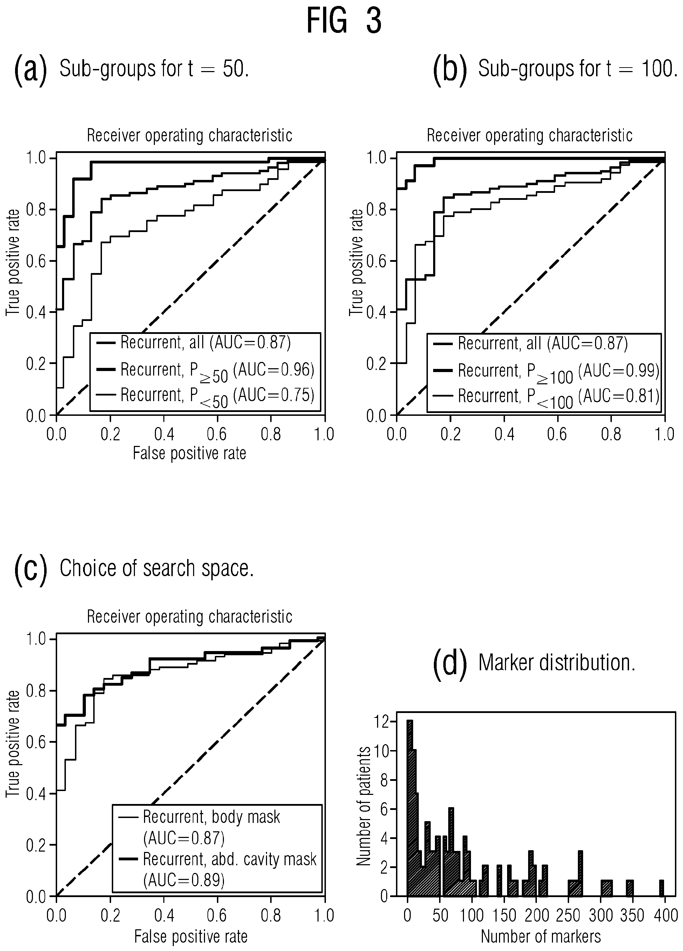

The difficulty of the detection task varies substantially; the largest air volumes are immediately obvious even to a layperson, whereas a single, tiny bubble of non-physiologic air in the abdomen might be the proverbial needle in a haystack. In order to get a better intuition of the level of heterogeneity and how it affects the model performance, a simple sub-group analysis is employed: the number of markers placed per patient is treated as a surrogate for the amount of air, seeing as large air volumes are somewhat densely covered with markers to ensure that all sliding windows are properly labeled. Hence, the Nair pneumoperitoneum data sets are sorted with regard to the number of markers and then split into two partitions: P<t (P≥t) with less than (at least) t markers for t ∈ {50, 100}. The sizes of the sub-groups are |P<50| = 49, |P≥50| = 61 and |P<100| = 76, |P≥100| = 34. For evaluation of the individual sub-groups, each is separately pooled with all control cases for a robust assessment. See Fig. 3d for a histogram of the marker distribution.

### Evaluation

Table 1 shows the areas under the curve (AUC) for the ROC curves of the baseline, multi-scale and recurrent variants for case-level and window-level evaluations. On the window level, there is a continuous improvement from the baseline to the multi-scale to the recurrent variant. While the changes may seem minimal, the difference between the baseline and the recurrent variant is in fact statistically significant with p < 10-4 in a two-tailed paired-sample t-test over the CV repetitions. This can be explained by the fact that the AUC is highly reproducible, as reflected by the small standard deviations, due to the large total number of patches. However, it can also be seen that this improvement does not translate directly to the case-level. Here, the AUC even decreases a little for the enhanced variants. However, all of these differences are statistically insignificant: The three models can be assumed to perform equally well in the case-level evaluation. In essence, this means that the patches that were classified more accurately by the enhanced models were not influential for the case-level decisions, at least in the limited number of cases available. For the sake of conciseness, the remaining results are shown only for the best-performing, recurrent model.

**Table 1: AUC values for window-level and case-level evaluation, given in percent as mean ± standard deviation over five random repetitions of five-fold CV.**

| Evaluation | Baseline | Multi-scale | Recurrent |
|---|---|---|---|
| Window level | 94.8±0.20 | 95.0±0.5% | 95.6±0.2% |
| Case level | 86.2±1.50 | 84.8 ±2.8% | 85.3 ± 2.2 % |

In Figs. 3a through 3c, ROC curves for one exemplary CV run are shown. Figs. 3a and 3b summarize the results of the sub-group analysis. For the groups P≥50 and P≥100, AUCs of 96 % and 99 % are achieved even though these still contain 55 % and 31 % of all data sets, respectively. In other words, while there are very difficult cases that limit the system's overall sensitivity, many of the pneumoperitoneum cases can be classified almost perfectly. In this context, it should be noted that although any amount of free intra-abdominal air can indicate an emergency, there is evidence that local oc-curences are clinically less significant [3]. Fig. 3c shows the change in performance when using the abdominopelvic cavity segmentation instead of the body mask, which suggests that elimination of candidates outside of the abdomen is beneficial.

### Alternative Approaches

Besides the presented mode of annotation where markers are placed inside the free intra-abdominal air regions, others which might lend themselves better to training a detection model were originally considered as well. Specifically, at first glance it may seem trivial to obtain a dense, pixel-wise labeling of the regions, seeing as air in CT can usually be segmented even by mere thresholding. This appears particularly promising as such ground truth masks would enable the use of successful image-to-image segmentation architectures [11], which-for good reasons-make up the majority of the current state of the art. This may be problematic for two reasons. First, soft reconstruction kernels and limited image resolution can cause the bowel walls to become partially invisible, obscuring the transition from intra- to extraluminal space.

Second, and more crucially, even with sophisticated segmentation tools it would be prohibitively time-consuming to segment all individual air bubbles in a patient, which can number in the dozens and may be interspersed with physiological air bubbles that prevent one from simply marking all air in a given region. In contrast, in the present approach, only some representative bubbles need to be marked to obtain correct labels for each patch. An annotation of bounding boxes, which could be used to train region proposal networks or similar modern detection models [16], did not seem feasible either. This is in large part due to the aforementioned huge number of tiny, distributed air bubbles for which it would be difficult to decide how to cover them with boxes in a consistent manner, whereas huge air volumes can sometimes occupy about half of the patient's abdomen.

The method of the invention follows a sliding-window approach with a deep recurrent neural network classifier at its core. When relying on an abdominopelvic cavity segmentation, it achieves a ROC AUC of 89 % in a five-fold CV. High specificity can be achieved while maintaining reasonable detection rates. Such an operating point is suitable for adjusting the reading order of emergency scans, where an unwarranted prioritization pre-empting more urgent cases is typically considered more harmful than a missed opportunity for earlier detection. This is particularly relevant as less than 1 % of emergency patients present with pneumoperitoneum [9].

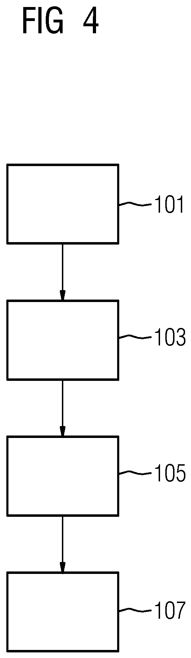

Fig. 4 shows a schematic representation of an embodiment of the method of the invention wherein input data is received 101, via a first interface. The input data said comprises a medical imaging data set of an abdominal region of a patient. Further, the input data is 3D imaging data and the 3D imaging data is subdivided 103 into a plurality of image patches of a preselected size. Then, a trained function is applied 105 and the output data is generated. The output data is provided 107 e.g. via a second interface. Outputting 107 can be achieved via a dedicated interface and the determined output data can thus be provided to a display, a printer, a memory, a network or in any other fashion suitable for inspection by a user, storage or further processing. The determined output data is indicative of a presence or absence of free intra-abdominal air. The output data can comprise a case-level information, indicating presence or absence of free intra-abdominal air for the medical imaging data set as a whole. Alternatively or additionally, the output data can provide a probability or score indicating the likelihood of presence or absence of free intra-abdominal air. Alternatively or additionally, the output data can provide the same types of information on the level of individual image patches or sub-regions. Alternatively or additionally, the output data can provide further information related to free intra-abdominal air, such as a volume of free intra-abdominal air, a location of a region of free intra-abdominal air relative to anatomical landmarks and similar information.

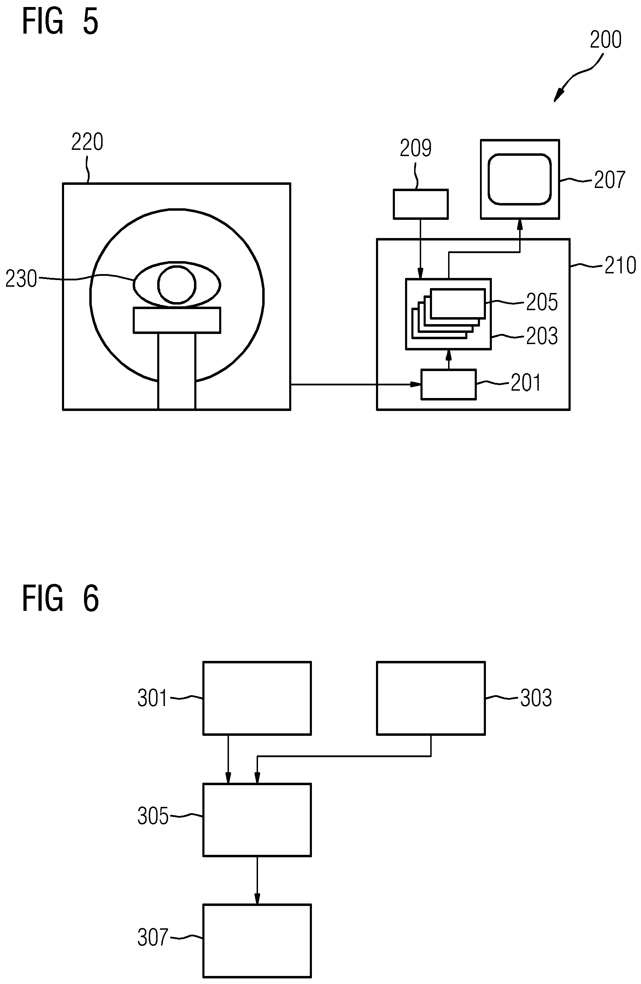

Fig. 5 shows a schematic representation of an embodiment of the system of the invention 200. This exemplary embodiment comprises a control unit 210 and a CT scanner 220 for scanning a patient 230. The control unit comprises a first interface 201 for receiving input data comprising a medical image data set. The control unit 210 further comprises a computation unit 203, which may optionally comprise a plurality of distinct further calculation units 205. Further there is provided a second interface 207 for outputting output data, and a command and data input interface 209 for inputting commands, such as selecting commands, control commands and other commands or data by a user.

Fig. 6 shows a schematic representation of an embodiment of the training method of the invention with the steps of receiving first input training data 301 (e.g. with a first training interface) comprising a plurality of medical imaging data sets of an abdominal region without free intra-abdominal air; receiving second input training data 303(e.g. with a second training interface), wherein image regions containing free intra-abdominal air are present and have been annotated; training a function 305 based on the first input training data and the second input training data (e.g. with a training computation unit); and providing 307 the trained function (e.g. with a third training interface). Outputting 307 can be achieved via a dedicated interface and the trained function can thus be provided to a display, a printer, a memory, a network or in any other fashion suitable for inspection by a user, storage or further processing.

By using the method and system of the invention an early detection of pneumoperitoneum is supported, and there will be fewer patients for whom pneumoperitoneum is detected later than it could have been or even missed entirely.

The detection is preferably automatic and can be performed at the medical imaging scanner. This means that patients with a suspicion of pneumoperitoneum can be prioritized without manual interaction. The automatic detection accuracy can be improved by using the best-suited images, and clinicians/radiologists can be notified immediately after the scan.

This provides an improved solution for emergency CT imaging, thereby providing a smart, "emergency-ready" CT scanner.

Applying modern machine learning techniques to the problem at hand, in particular deep learning which is able to derive its own features, is key as the task of identifying intra-abdominal free air requires a comparatively high degree of anatomical understanding which is much harder to attain with traditional methods where such an understanding must typically be achieved with hand-crafted features.

The exemplary embodiments have been described for illustrative purposes. It will be obvious that the described features, steps and workflow may be varied in many ways. Such variations are not to be regarded as a departure from the scope of the present invention, and all such modifications as would be obvious to one skilled in the art are intended to be included within the scope of the following claims.

### References

1. Bulas, D.I., Taylor, G.A., Eichelberger, M.R.: The value of ct in detecting bowel perforation in children after blunt abdominal trauma. American Journal of Roentgenology 153(3), 561-564 (1989)
2. Cho, K., Van Merri¨enboer, B., Gulcehre, C., Bahdanau, D., Bougares, F., Schwenk, H., Bengio, Y.: Learning phrase representations using rnn encoder-decoder for statistical machine translation. arXiv preprint arXiv:1406.1078 (2014)
3. Cho, S.J., Choi, I.J., Kim, S.J., Kim, M.J., Kim, C.G., Lee, J.Y., Ryu, K.W., Kim, Y.W.: Clinical significance of intraperitoneal air on computed tomography scan after endoscopic submucosal dissection in patients with gastric neoplasms. Surgical endoscopy 28(1), 307-313 (2014)
4. Fawcett, T.: An introduction to ROC analysis. Pattern recognition letters 27(8), 861-874 (2006)
5. Hainaux, B., Agneessens, E., Bertinotti, R., De Maertelaer, V., Rubesova, E., Capelluto, E., Moschopoulos, C.: Accuracy of mdct in predicting site of gastroin- testinal tract perforation. American Journal of Roentgenology 187(5), 1179-1183 (2006)
6. Kingma, D.P., Ba, J.: Adam: A method for stochastic optimization. arXiv preprint arXiv:1412.6980 (2014)
7. Luo, J.W., Lie, J.L., Chong, J.: Pneumoperitoneum on chest X-ray: A DCNN approach to automated detection and localization utilizing salience and class activation maps. In: SIIM Conference on Machine Intelligence in Medical Imaging (2018)
8. Matthews, B.W.: Comparison of the predicted and observed secondary structure of t4 phage lysozyme. Biochimica et Bio-physica Acta (BBA)-Protein Structure 405(2), 442-451 (1975)
9. Nazerian, P., Tozzetti, C., Vanni, S., Bartolucci, M., Gualtieri, S., Trausi, F., Vittorini, M., Catini, E., Cibi-nel, G.A., Grifoni, S.: Accuracy of abdominal ul- trasound for the diagnosis of pneumoperitoneum in patients with acute ab- dominal pain: a pilot study. Critical Ultrasound Journal 7(1), 15 (2015). https://doi.org/10.1186/s13089-015-0032-6
10. Paster, S.B., Brogdon, B.G.: Roentgenographic Diagnosis of Pneumoperitoneum. JAMA 235(12), 1264-1267 (03 1976). https://doi.org/10.1001/jama.1976.03260380058035
11. Ronneberger, O., Fischer, P., Brox, T.: U-net: Convolutional networks for biomedical image segmentation. In: International Conference on Medical image computing and computer-assisted intervention. pp. 234-241. Springer (2015)
12. Shi, B., Bai, X., Yao, C.: An end-to-end trainable neural network for image-based sequence recognition and its application to scene text recognition. IEEE Transac- tions on Pattern Analysis and Machine Intelligence, 2016
13. Summers, R.M.: Progress in fully automated abdominal CT interpretation. Amer- ican Journal of Roentgenology 207(1), 67-79 (2016)
14. Tompson, J.J., Jain, A., LeCun, Y., Bregler, C.: Joint training of a convolutional network and a graphical model for human pose estimation. In: Advances in neural information processing systems. pp. 1799-1807 (2014)
15. Yang, D., Xu, D., Zhou, S.K., Georgescu, B., Chen, M., Grbic, S., Metaxas, D., Comaniciu, D.: Automatic liver segmentation using an adversarial image-to-image network. In: International Conference on Medical Image Computing and Computer-Assisted Intervention. pp. 507-515. Springer (2017)

## Claims

1. A method for the detection of free intra-abdominal air, comprising:
- receiving input data, said input data comprising a medical imaging data set of an abdominal region of a patient,
- applying a trained function, wherein the output data is generated,
- providing the output data e.g. via a second interface, wherein the trained function is trained on training data comprising a plurality of medical imaging data sets of an abdominal region, wherein image regions containing free intra-abdominal air are annotated,
wherein the medical imaging data set comprises 3D imaging data and wherein the 3D imaging data is subdivided into a plurality of image patches of a preselected size, wherein the trained function is used to determine whether any given image patch the plurality of image patches contains free intra-abdominal air, and
wherein a classification information or score or probability information is determined for each image patch.

2. The method according to claim 1 wherein the annotation of an image region containing free intra-abdominal air comprises placing a marker manually that indicates a position inside a region of free intra-abdominal air.

3. The method according to any of the previous claims wherein the trained function is based on a convolutional neural network or deep neural network.

4. A system or apparatus for the detection of free intra-abdominal air according to the method of any of claims 1 to 3, comprising
- a first interface, configured for receiving input data,
- a second interface, configured for providing output data,
- a computation unit, configured for applying a trained function, wherein the output data is generated.

5. A computer program comprising instructions which, when the program is executed by a computer, carries out the method of one of the claims 1 to 3.

6. A computer-readable medium comprising instructions to carry out the method of one of the claims 1 to 3 when executed by a computer.

7. A method for providing a trained function for the detection of free intra-abdominal air, comprising:
- receiving first input training data comprising a first plurality of medical imaging data sets of an abdominal region, wherein the medical imaging data sets of the first plurality of medical imaging data sets do not comprise image regions containing free intra-abdominal air,
- receiving second input training data comprising a second plurality of medical imaging data sets of an abdominal region, wherein each medical imaging data set of the second plurality of medical imaging data sets comprises at least one image region containing free intra-abdominal air,
- training a function based on the input training data and the out-put training data,
- providing the trained function,
wherein the medical imaging data sets of the first and second input training data comprise 3D imaging data and wherein the 3D imaging data is subdivided into a plurality of image patches of a preselected size,
wherein the trained function is trained to determine whether any given image patch the plurality of image patches contains free intra-abdominal air, and
wherein a classification information or score or probability information may be determined for each image patch.

8. The method according to claim 7, wherein the trained function is based on a convolutional neural network or deep neural network.

9. The method according to claim 7 or 8, wherein each medical imaging data set of the second plurality of medical imaging data sets comprises annotation information indicating the at least one image region containing free intra-abdominal air.

10. A training system for training a function for the detection of free intra-abdominal air provided by the method of any of claims 7 to 9, comprising
- a first training interface, configured for receiving input training data,
- a second training interface, configured for receiving output training data, wherein the input training data is related to the output training data,
- a training computation unit, configured training a function based on the input training data and the out-put training data,
- a third training interface, configured for providing the trained function.

11. A computer program comprising instructions which, when the program is executed by a computer, cause the computer to carry out the method of one of the claims 8 to 9.

12. A computer-readable medium comprising instructions which, when executed by a computer, cause the computer to carry out the method of one of the claims 8 to 9.

## Patentansprüche

1. Verfahren zur Detektion von freier intraabdominaler Luft, umfassend:
- Empfangen von Eingabedaten, wobei die Eingabedaten einen medizinischen Bildgebungsdatensatz einer Abdominalregion eines Patienten umfassen,
- Anwenden einer trainierten Funktion, wobei die Ausgabedaten generiert werden,
- Bereitstellen der Ausgabedaten z. B. über eine zweite Schnittstelle,
wobei die trainierte Funktion an Trainingsdaten trainiert wird, die mehrere medizinische Bildgebungsdatensätze einer Abdominalregion umfassen, wobei Bildregionen, welche freie intraabdominale Luft enthalten, annotiert werden,
wobei der medizinische Bildgebungsdatensatz 3D-Bildgebungsdaten umfasst und wobei die 3D-Bildgebungsdaten in eine Mehrzahl von Bildpatches einer vorab ausgewählten Größe unterteilt werden,
wobei die trainierte Funktion verwendet wird, um zu bestimmen, ob irgendein gegebener Bildpatch der Mehrzahl von Bildpatches freie intraabdominale Luft enthält, und
wobei Klassifizierungsinformationen oder eine Bewertung oder Wahrscheinlichkeitsinformationen für jeden Bildpatch bestimmt werden.

2. Verfahren gemäß Anspruch 1, wobei die Annotation einer Bildregion, welche freie intraabdominale Luft enthält, das manuelle Platzieren eines Markers umfasst, der eine Position innerhalb einer Region von freier intraabdominaler Luft angibt.

3. Verfahren gemäß einem der vorhergehenden Ansprüche,
wobei die trainierte Funktion auf einem faltenden neuronalen Netz oder einem tiefen neuronalen Netz basiert.

4. System oder Einrichtung zur Detektion von freier intraabdominaler Luft gemäß dem Verfahren nach einem der Ansprüche 1 bis 3, umfassend
- eine erste Schnittstelle, die zum Empfangen von Eingabedaten ausgelegt ist,
- eine zweite Schnittstelle, die zum Bereitstellen von Ausgabedaten ausgelegt ist,
- eine Recheneinheit, die zum Anwenden einer trainierten Funktion ausgelegt ist, wobei die Ausgabedaten generiert werden.

5. Computerprogramm, Anweisungen umfassend, das bei Ausführung des Programms durch einen Computer das Verfahren nach einem der Ansprüche 1 bis 3 durchführt.

6. Computerlesbares Medium, umfassend Anweisungen zum Ausführen des Verfahrens nach einem der Ansprüche 1 bis 3 bei Ausführung durch einen Computer.

7. Verfahren zum Bereitstellen einer trainierten Funktion zur Detektion von freier intraabdominaler Luft, umfassend:
- Empfangen von ersten Trainingseingabedaten, umfassend eine erste Mehrzahl von medizinischen Bildgebungsdatensätzen einer Abdominalregion, wobei die medizinischen Bildgebungsdatensätze der ersten Mehrzahl von medizinischen Bildgebungsdatensätzen keine Bildregionen umfassen, die freie intraabdominale Luft enthalten,
- Empfangen von zweiten Trainingseingabedaten, umfassend eine zweite Mehrzahl von medizinischen Bildgebungsdatensätzen einer Abdominalregion, wobei jeder medizinische Bildgebungsdatensatz der zweiten Mehrzahl von medizinischen Bildgebungsdatensätzen wenigstens eine Bildregion umfasst, die freie intraabdominale Luft enthält,
- Trainieren einer Funktion basierend auf den Trainingseingabedaten und den Trainingsausgabedaten,
- Bereitstellen der trainierten Funktion,
wobei die medizinischen Bildgebungsdatensätze der ersten und der zweiten Trainingseingabedaten 3D-Bildgebungsdaten umfassen und wobei die 3D-Bildgebungsdaten in eine Mehrzahl von Bildpatches einer vorab ausgewählten Größe unterteilt sind,
wobei die trainierte Funktion dazu trainiert ist zu bestimmen, ob irgendein gegebener Bildpatch der Mehrzahl von Bildpatches freie intraabdominale Luft enthält, und
wobei Klassifizierungsinformationen oder eine Bewertung oder Wahrscheinlichkeitsinformationen für jeden Bildpatch bestimmt werden können.

8. Verfahren gemäß Anspruch 7, wobei die trainierte Funktion auf einem faltenden neuronalen Netz oder einem tiefen neuronalen Netz basiert.

9. Verfahren gemäß Anspruch 7 oder 8, wobei jeder medizinische Bildgebungsdatensatz der zweiten Mehrzahl von medizinischen Bildgebungsdatensätzen Annotationsinformationen umfasst, welche die wenigstens eine Bildregion angeben, die freie intraabdominale Luft enthält.

10. Trainingssystem zum Trainieren einer Funktion zur Detektion von freier intraabdominaler Luft, bereitgestellt durch das Verfahren nach einem der Ansprüche 7 bis 9, umfassend
- eine erste Trainingsschnittstelle, die zum Empfangen von Trainingseingabedaten ausgelegt ist,
- eine zweite Trainingsschnittstelle, die zum Empfangen von Trainingsausgabedaten ausgelegt ist, wobei die Trainingseingabedaten mit den Trainingsausgabedaten in Beziehung stehen,
- eine Trainingsrecheneinheit, die zum Trainieren einer Funktion basierend auf den Trainingseingabedaten und den Trainingsausgabedaten ausgelegt ist,
- eine dritte Trainingsschnittstelle, die zum Bereitstellen der trainierten Funktion ausgelegt ist.

11. Computerprogramm, umfassend Anweisungen, die bei Ausführung des Programms durch einen Computer den Computer veranlassen, das Verfahren nach einem der Ansprüche 8 bis 9 durchzuführen.

12. Computerlesbares Medium, umfassend Anweisungen, die bei Ausführung durch einen Computer den Computer veranlassen, das Verfahren nach einem der Ansprüche 8 bis 9 auszuführen.

## Revendications

1. Un procédé de détection d'air intra-abdominal, comprenant :
- recevoir une donnée d'entrée, ladite donnée d'entrée comprenant un ensemble de données d'imagerie médicale d'une région abdominale d'un patient,
- appliquer une fonction entraînée, la fonction de sortie étant créée,
- se procurer la donnée de sortie, par exemple par l'intermédiaire d'une deuxième interface, dans lequel la fonction entraînée est entraînée sur des données d'entraînement comprenant une pluralité d'ensembles de données d'imagerie médicale d'une région abdominale, dans lequel les régions d'image contenant de l'air intra-abdominal libre sont annotées,
dans lequel l'ensemble de données d'imagerie médicale comprend une donnée d'imagerie en 3D, dans lequel la donnée d'imagerie en 3D est subdivisée en une pluralité de pièces d'image d'une dimension sélectionnée à l'avance,
dans lequel la fonction entraînée est utilisée pour déterminer si une pièce d'image donnée de la pluralité de pièces d'image contient de l'air intra-abdominal libre, dans lequel une information de classement ou un score ou une information de probabilité est déterminée pour chaque pièce d'image.

2. Le procédé suivant la revendication 1, dans lequel l'annotation d'une région d'image contenant de l'air intra-abdominal libre comprend mettre manuellement un marqueur, qui indique une position à l'intérieur d'une région d'air intra-abdominal libre.

3. Le procédé suivant l'une quelconque des revendications précédentes, dans lequel la fonction entraînée repose sur un réseau neuronal de convolution ou sur un réseau neuronal profond.

4. Un système ou un dispositif de détection d'air intra-abdominal libre suivant le procédé de l'une quelconque des revendications 1 à 3, comprenant
- une première interface, configurée pour recevoir une donnée d'entrée,
- une deuxième interface, configurée pour donner une donnée de sortie,
- une unité informatique, configurée pour appliquer une fonction entraînée, la donnée de sortie étant créée.

5. Un programme d'ordinateur comprenant des instructions qui, lorsque le programme est exécuté par un ordinateur, exécutent le procédé suivant l'une des revendications 1 à 3.

6. Un support, déchiffrable par ordinateur, comprenant des instructions pour effectuer le procédé de l'une des revendications 1 à 3, lorsqu'elles sont exécutées par un ordinateur.

7. Un procédé pour donner une fonction entraînée pour la détection d'air intra-abdominal libre, comprenant :
- recevoir une première donnée d'entraînement d'entrée comprenant une première pluralité d'ensembles de données d'imagerie médicale d'une région abdominale, dans lequel les ensembles de données d'imagerie médicale de la première pluralité d'ensembles de données d'imagerie médicale ne comprennent pas des régions d'image contenant de l'air intra-abdominal libre,
- recevoir une deuxième donnée d'entraînement d'entrée comprenant une deuxième pluralité d'ensembles de données d'imagerie médicale d'une région abdominale, dans lequel chaque ensemble de données d'imagerie médicale de la deuxième pluralité d'ensembles de données d'imagerie médicale comprend au moins une région d'image contenant de l'air intra-abdominal libre,
- entraîner une fonction reposant sur la donnée d'entrée d'entraînement et la donnée de sortie d'entraînement,
- donner la fonction entraînée,
dans lequel les ensembles de données d'imagerie médicale de la première et de la deuxième données d'entrée d'entraînement comprennent une donnée d'imagerie en 3D, et dans lequel la donnée d'imagerie en 3D est subdivisée en une pluralité de pièces d'image d'une dimension sélectionnée à l'avance,
dans lequel la fonction entraînée est entraînée pour déterminer si une pièce d'image donnée de la pluralité de pièces d'image comprend de l'air intra-abdominal libre, et dans lequel une information de classement ou un score ou une information de probabilité peut être déterminé pour chaque pièce d'image.

8. Un procédé suivant la revendication 7, dans lequel la fonction entraînée repose sur un réseau neuronal de convolution ou sur un réseau neuronal profond.

9. Un procédé suivant la revendication 7 ou 8, dans lequel chaque ensemble de données d'imagerie médicale de la deuxième pluralité d'ensembles de données d'imagerie médicale comprend une information d'annotation indiquant la au moins une région d'image contenant de l'air intra-abdominal libre.

10. Un système d'entraînement pour entraîner une fonction de détection d'air intra-abdominal libre donné par le procédé de l'une quelconque des revendications 7 à 9, comprenant
- une première interface d'entraînement, configurée pour recevoir une donnée d'entrée d'entraînement,
- une deuxième interface d'entraînement, configurée pour recevoir une donnée de sortie d'entraînement, la donnée d'entrée d'entraînement étant en relation avec la donnée de sortie d'entraînement,
- une unité informatique d'entraînement, configurée pour entraîner une fonction reposant sur la donnée d'entrée d'entraînement et la donnée de sortie d'entraînement,
- une troisième interface d'entraînement, configurée pour donner la fonction entraînée.

11. Un programme d'ordinateur comprenant des instructions qui, lorsque le programme est exécuté par ordinateur, font que l'ordinateur exécute le procédé suivant l'une des revendications 8 à 9.

12. Un support, déchiffrable par ordinateur, comprenant des instructions qui, lorsqu'elles sont exécutées par un ordinateur, font que l'ordinateur exécute le procédé suivant l'une des revendications 8 à 9.
